# EUROPÄISCHE PATENTSCHRIFT

(11) **EP 3 940 080 B1**
(45) Veröffentlichungstag und Bekanntmachung des Hinweises auf die Patenterteilung: **02.04.2025**
(21) Anmeldenummer: 20186570.6
(22) Anmeldetag: 17.07.2020
(51) Int. Cl.: C12Q 1/00, C12Q 1/02, G01N 33/50, G01N 33/52, G01N 30/90

(54) **ERFINDUNG BETREFFEND ANALYSE VON MINDESTENS EINER CHEMISCHEN VERBINDUNG DURCH SIMULATION MINDESTENS EINES METABOLISIERUNGSVORGANGES**
INVENTION RELATING TO THE ANALYSIS OF AT LEAST ONE CHEMICAL COMPOUND BY SIMULATING AT LEAST ONE METABOLISATION PROCESS
INVENTION CONCERNANT L'ANALYSE D'AU MOINS UN COMPOSÉ CHIMIQUE PAR SIMULATION D'AU MOINS UN PROCESSUS DE MÉTABOLISATION

(43) Veröffentlichungstag der Anmeldung: 19.01.2022
(73) Patentinhaber: Justus-Liebig-Universität Gießen, Körperschaft des öffentlichen Rechts, 35390 Gießen (DE)
(72) Erfinder: Morlock, Gertrud, 35392 Gießen (DE); Klingelhöfer, Ines, 35415 Pohlheim (DE)
(74) Vertreter: Stumpf, Peter

(56) Entgegenhaltungen:
- EP-A1- 2 919 004
- SEBASTIAN BUCHINGER ET AL: "Direct Coupling of Thin-Layer Chromatography with a Bioassay for the Detection of Estrogenic Compounds: Applications for Effect-Directed Analysis", ANALYTICAL CHEMISTRY, vol. 85, no. 15, 24 July 2013 (2013-07-24), pages 7248 - 7256, XP055744799, ISSN: 0003-2700, DOI: 10.1021/ac4010925
- MARYAM JAMSHIDI-AIDJI ET AL: "Effect-directed profiling of aqueous, fermented plant preparations via high-performance thin-layer chromatography combined with in situ assays and high-resolution mass spectrometry", JOURNAL OF LIQUID CHROMATOGRAPHY AND RELATED TECHNOLOGIES, vol. 42, no. 9-10, 2 April 2019 (2019-04-02), US, pages 266 - 273, XP055744802, ISSN: 1082-6076, DOI: 10.1080/10826076.2019.1585631
- GUIYANG LIU ET AL: "Effects of bifidobacteria-produced exopolysaccharides on human gut microbiota in vitro", APPLIED MICROBIOLOGY AND BIOTECHNOLOGY, vol. 103, no. 4, 19 December 2018 (2018-12-19), Berlin/Heidelberg, pages 1693 - 1702, XP055744772, ISSN: 0175-7598, DOI: 10.1007/s00253-018-9572-6
- SHAOFENG BAI ET AL: "Comparative study on the in vitro effects of Pseudomonas aeruginosa and seaweed alginates on human gut microbiota", PLOS ONE, vol. 12, no. 2, 22 February 2017 (2017-02-22), pages e0171576, XP055744721, DOI: 10.1371/journal.pone.0171576
- RICHARD HORNIBLOW ET AL: "The Safety and Tolerability of a Potential Alginate-Based Iron Chelator; Results of A Healthy Participant Study", NUTRIENTS, vol. 11, no. 3, 21 March 2019 (2019-03-21), pages 674, XP055745135, DOI: 10.3390/nu11030674
- LORENZO NISSEN ET AL: "ABSTRACT", FEMS MICROBIOLOGY LETTERS, vol. 367, no. 12, 1 June 2020 (2020-06-01), XP055745131, DOI: 10.1093/femsle/fnaa097
- GERTRUD E. MORLOCK: "Miniaturized planar chromatography using office peripherals - Office chromatography", JOURNAL OF CHROMATOGRAPHY A, vol. 1382, 1 February 2015 (2015-02-01), AMSTERDAM, NL, pages 87 - 96, XP055745104, ISSN: 0021-9673, DOI: 10.1016/j.chroma.2014.09.050
- BRODKORB ANDRÉ ET AL: "INFOGEST static in vitro simulation of gastrointestinal food digestion", NATURE PROTOCOLS, NATURE PUBLISHING GROUP, GB, vol. 14, no. 4, 18 March 2019 (2019-03-18), pages 991 - 1014, XP037471310, ISSN: 1754-2189, [retrieved on 20190318], DOI: 10.1038/S41596-018-0119-1

## Beschreibung

Die vorliegende Erfindung betrifft die Simulation mindestens eines Metabolisierungsvorganges in miniaturisierter Form in situ zur Analyse einer bekannten oder unbekannten Probe mit mindestens einer darin enthaltenen chemischen Verbindung. Somit ist es möglich, in einem einzigen all-in-one System (Reaktion/Trennung/Detektion/Wirkung) die Wirkung von Metabolisierungsvorgängen auf verschiedene chemische Verbindungen oder den Einfluss von chemischen Verbindungen auf Metabolisierungsvorgänge zu analysieren.

Ein Metabolisierungsvorgang umfasst dabei einen Vorgang aus dem Stoffwechsel oder Metabolismus eines Organismus. Dies sind chemische oder physikalische Vorgänge der Umsetzung chemischer Stoffe bzw. Substrate in Zwischenprodukte (Metaboliten) und Endprodukte im Organismus. Ein typischer Metabolisierungsvorgang wird mit Enzymen umgesetzt, die verschiedene chemische Reaktionen beschleunigen und katalysieren. Metabolisierungsvorgänge sind aus allen Organismen bekannt. Bekannte Metabolisierungsvorgänge von Wirbeltieren sind der Verdauungstrakt mit Mund-, Magen- und Darmbereich sowie der Gastrointestinalbereich (GIT) dar. Alle Vorgänge und Organismen sind in einem Mikrobiom-System zusammengefasst und für jeden Organismus bzw. jede Art spezifisch.

### Stand der Technik

Da das Mikrobiom von Menschen und Wirbeltieren für die medizinische Therapie und das Verständnis eines gesunden Lebens immer wichtiger wird, ist es unerlässlich, Wirkstoffe in der Nahrung oder im Futter zu identifizieren, die erst durch Verdau im Magen-Darm-Trakt bzw. ihrer Verstoffwechselung gebildet werden. Es fehlen jedoch bioanalytische bildgebende Verfahren, die in der Lage sind, Lebens- und Futtermittel umfassend auf ihre Wirkung nach der Verstoffwechslung zu untersuchen und ihre Komplexität in ein leicht verständliches Ergebnis, z.B. bildliche Darstellung oder Chromatogramm, zu übersetzen. Um z.B. die Aktivität eines Stoffgemisches auf den menschlichen oder tierischen Verdauungstrakt zu testen sind bislang mehrere aufeinanderfolgende Verfahrensschritte erforderlich, z.B. die Trennung des Stoffgemisches und anschließende mühsame Isolierung der einzelnen Stoffe und Verbindungen sowie die Testung dieser isolierten Einzelstoffe im separaten Verdausystem, gefolgt von einem chromatographischen System zur Erfassung der Veränderungen. Meist ist zusätzlich ein Konzentrationsschritt in ein geeignetes Lösungsmittel erforderlich.

Die Testung von Metabolisierungsvorgängen auf Lebensmittel, Futtermittel, Arzneistoffe oder sonstige chemische Verbindungen ist entweder langwierig, teuer, kontaminations- und fehleranfällig oder in speziellen Fällen überhaupt nicht möglich. Die einzelnen Verfahrensschritte müssen zudem von Fachpersonal durchgeführt und ausgewertet werden. Die Simulation von menschlichen oder tierischen Verdauungssystem ist besonders aufwendig und schwierig, da die vielfältigen Vorgänge aus Enzymen, Bakterien und Zellbestandteilen des Mikrobioms berücksichtigt werden müssen. Bei den menschlichen oder tierischen Verdausystemen unterscheidet man dynamische und statische Systeme. Aufwendiger und teurer sind besonders die im Stand der Technik bekannten, dynamischen Simulationsmodelle, da hier z.B. mit immobilisierten Enzymen in einer oder mehreren hintereinandergeschalteten Säulen gearbeitet wird, die mit ca. 200 000 Euro extrem teuer sind, so dass sie nicht für alle Fragestellungen oder Routineanwendungen verwendet werden. Dies erklärt die bisher nur rudimentäre Forschung zu Metabolisierungsvorgängen.

Zur Trennung eines Stoffgemisches aus chemischen Verbindungen kennt der Fachmann Adsorbentien auf Kieselgel- oder Polymerbasis, die mit diversen funktionellen Gruppen belegt sein können und eine hohe spezifische Oberfläche und gewisse Porosität (auch Durchdringbarkeit) aufweisen. Adsorbentien sind Stoffe, die Gase und gelöste Substanzen physico-chemisch binden, also adsorbieren können. Bei planaren Trennungen in der Dünnschichtchromatografie (DC) oder Hochleistungsdünnschichtchromatographie (*HPTLC,* von engl. high-performance thin-layer chromatography) wird bevorzugt Kieselgel als Adsorptionsmaterial verwendet, um Stoffgemische aufzutrennen. Kieselgel (auch Kieselsäuregel, Kieselgur, Silikagel, silica gel) ist ein farbloses, amorphes Siliciumdioxid. Es weist eine gelartige, gummiartige bis feste partikuläre, sphärische oder monolithische Konsistenz auf. Es besitzt eine große innere Oberfläche (z.B. 350 oder 600 m²/g) und ist stark hygroskopisch (wasseranziehend). Es kann auch zusammen mit Polymeren als Hybridmaterial (auch elektroversponnen) vorkommen. Bevorzugt wird modifiziertes Kieselgel verwendet. Zum Beispiel ein wasserbenetzbares modifiziertes Kieselgel für die wirkungsbezogene Analytik. Dies hat den Vorteil, dass nachgeschaltete Bioassays auf wässriger Basis gut darauf aufzubringen sind und die Diffusion der Zonen limitiert ist. Weiterhin bekannt sind zweiphasige Trägermaterialien, die aus zwei verschiedenen Adsorbentien auf demselben Träger bestehen. Der Stand der Technik offenbart in Buchinger et al. (2013) ein Verfahren zum Nachweis der östrogenen Aktivität in Umweltproben (auch in kosmetischen Proben) basierend auf der Kombination von Hochleistungs-Dünnschichtchromatografie mit einem "in-situ" -Hefeöstrogentest (YES-Test) zur wirkungsbezogenen Analyse. Das Verfahren umfasst i) Abscheiden einer Probe mit einem komplexen Gemisch chemischer Substanzen auf einem (Kieselgel-) Träger für die Dünnschicht-chromatografie, ii) Chromatografische Trennung der auf dem Träger befundenen Substanzen, iii) Eintauchen der Platte in eine Zellsuspension und Besprühen der Platte mit einem Substrat für eine enzymatische Detektion, die zur Entstehung eines fluoreszierenden Produkts führt und iv) Bestimmung des östrogenen Profits der Probe durch Erfassung und Quantifizierung der Fluoreszenz.

M. Jamshidi-Aidji et al. (2019) offenbart ein ähnliches Verfahren zur Untersuchung möglicher therapeutischer Eigenschaften (z. B. als antibakterieller oder Sauerstofffänger) fermentierter Pflanzenpräparate unter Verwendung verschiedener (Bio-) Essays. Hier wird die Fermentationslösung einem chromatografischen Verfahren (Dünnschichtchromatografie) unterzogen, um verschiedene Metabolisierungsprodukte aus dem Fermentationsprozess zu trennen und diese anschließend durch Zugabe einer exogenen Beta-Galactosidase und eines geeigneten Substrats sichtbar zu machen. Ferner, werden die verschiedene Komponenten der Probe nach der chromatografischen Trennung mittels Massenspektrometrie validiert und quantifiziert.

Beide Schriften offenbaren keinen Stoffwechselvorgang des Mikrobiom-Systems der Verdauungstrakt von Wirbeltieren und die (chromatographische) Trennung findet erst nach dem Inkontaktbringen des Trägers mit dem Enzym/Zell-System statt. Der Stand der Technik kenn weiterhin G. Liu et al. (2018) und S. Bai et al. (2017) Beide offenbaren einen ähnlichen Ansatz zur Untersuchung der Wirkung von bakteriellen Exopolysacchariden in menschlichen Mikrobiota. Zu diesem Zweck werden Stuhlproben unter Verwendung der Exopolysaccharide als Kohlenstoffquelle "invitro" im Reagenzglas fermentiert. Die fermentierte Probe wird in einen Träger für die Dünnschichtchromatografie aufgetragen, wo die Produkte, die aus dem Abbau von Exopolysacchariden resultieren, abgetrennt und unter Verwendung eines geeigneten chemischen Reagens nachgewiesen werden.

R.D. Horniblow et al. (2019) offenbart die Wirkung von Eisen bei der Verarbeitung von Alginat in der Darmmikrobiota. Der Metabolisierungsvorgang wird mit der SHIME-Plattform simuliert und die Abbauprodukte von Alginat (ein Exopolysaccharide) werden mittels Dünnschichtchromatografie nachgewiesen.

A.Brodkorb et al. (2019) offenbart das "Infogest", ein Protokoll zur Simulation von Metabolisierungsvorgängen im Magen-Darm-Trakt im Labor. Die Produkte solcher Vorgänge werden im Anschluss analysiert.

Bei diesen Offenbarungen findet der Metabolisierungsvorgang vor dem Laden der Probe auf den chromatografischen Träger statt, weil die für den Stoffwechselprozess verantwortliche Enzyme/Zellen in die Probe enthalten sind.

Keines der Dokumente aus dem Stand der Technik offenbart ein Verfahren, bei dem der Träger mit der zu analysierenden Probe mit dem Enzym/Enzymsystem/Zellsystem vor der chromatographischer Trennung in Kontakt gebracht wird.

### Aufgabe

Aufgabe der vorliegenden Erfindung ist es ein vereinfachtes Verfahren zur Analyse von mindestens einer chemischen Verbindung einer Probe durch Simulation mindestens eines Metabolisierungsvorganges bereitzustellen, das zuverlässig durchgeführt werden kann.

### Lösung der Aufgabe

Die Lösung dieser Aufgabe ergibt sich aus den Merkmalen des Hauptanspruchs, während vorteilhafte Ausgestaltungen und Weiterbildungen der Erfindung den Unteransprüchen entnehmbar sind.

Es wird ein Verfahren zur Analyse von chemischen Verbindungen einer Probe bereitgestellt, wobei mindestens ein Metabolisierungsvorgang in miniaturisierter Form in situ simuliert wird, so dass die Wirkung von chemischen Verbindungen auch auf verschiedene Metabolisierungsvorgänge auf einem Träger analysiert werden können.

Dazu wird auf einem Träger, der ein bekanntes Trennmaterial umfasst, ein miniaturisiertes Metabolisierungssystem in situ simuliert. Damit können Probemengen im Pico-, Nano- bis hin zum Mikrogramm-Bereich sehr gut und zuverlässig untersucht werden.

Das Verfahren ermöglicht es, verschiedene Metabolisierungsvorgänge zu simulieren, eine chromatographische Trennung der Metabolisierungsprodukte sowie eine Mehrfach-Detektion (einschließlich der wirkungsbezogenen Detektion) der getrennten Metabolisierungsprodukte vorzunehmen, zur schnellen und zuverlässigen Erfassung von Veränderungen und /oder Effekten durch bioaktive Verbindungen, die durch bekannte Metabolisierungswege im menschlichen, tierischen, pflanzlichen oder pilzlichen Organismus erzeugt werden. Der Proof-of-Principle dieser Möglichkeiten wurde für grundlegende System z.B. Lebensmittelsysteme erbracht und diese finden sich in den Ausführungsbeispielen.

Die Simulation von Metabolisierungsvorgängen erfolgt z.B. durch Umsetzung mit mindestens einem Enzym, einem Enzymsystem und/oder mindestens einem tierischen oder pflanzlichen Zellsystem. Das Enzymsystem umfasst mindestens ein Enzym und den dazugehörenden Puffer. Das Zellsystem umfasst Suspensionszellen oder adhärente Zellen ausgewählt beispielsweise aus der Gruppe der Bakterien, Hefen, Pilze, Algen und Humanzellen, wie Blutzellen, Knochenzellen, Tumor-/Krebszellen, Darmzellen etc. Der Fachmann kennt viele Metabolisierungsvorgänge und die darin involvierten Enzyme, Stoffe und Rahmenbedingungen aus dem menschlichen oder tierischen Organismus, aus Pflanzen, Pilzen und Mikroorganismen. Beispielhaft wird das Verdauungssystem eines Menschen genannt, hier sind für den Metabolisierungsvorgang im Mundbereich Amylasen aktiv, im Magenbereich sind es vor allem Peptidasen/Pepsin und im Dünndarm Pankreatin und/oder Gallensalze. Ein typischer Metabolisierungsvorgang umfasst die Lyse.

Im erfindungsgemäßen Verfahren kann der mindestens eine Metabolisierungsvorgang prä- sowie postchromatografisch angewandt werden, vor und/oder nachdem die chemischen Verbindungen einer unbekannten Probe chromatografisch auf einem geeigneten Träger getrennt wurden. Die simulierten Metabolisierungsvorgänge können auf demselben Träger durchgeführt werden. Es finden zuerst die simulierten Metabolisierungsvorgänge auf dem Träger statt und anschließend erfolgt die chromatografische Trennung der metabolisierten Produkte auf demselben Träger. Alternativ erfolgt die chromatografische Trennung der Probe und danach erfolgt die simulierte Metabolisierung.

Die chromatografisch getrennten Verbindungen können anschließend auf demselben Träger detektiert werden und hinsichtlich Ihrer Wirkung in chemischen/biochemischen/biologischen Assays analysiert werden. Der Fachmann kennt verschiedene chemische Assays z.B. den DPPH•-Radikalfängerassay, er kennt auch verschiedene biochemische Assays wie z.B. den α-Amylase-Inhibitor-Assay, den Assay für AChE-Hemmstoffe oder Lipasehemmer. Bei den biologischen Assays kennt der Fachmann z.B. Östrogen-Assays, Androgen-Assays, antimikrobielle und genotoxische Assays. Diese können alleine oder in Kombination auf dem Träger eingesetzt werden.

Der Vorteil ist, dass nun auf einem Träger die Situation mindestens eines Metabolisierungsvorganges simuliert werden kann und zudem entstandene Produkte getrennt und detektiert werden können. Auf demselben Träger kann durch den Vergleich vor und nach der Metabolisierung ein Rückschluss auf Wirksamkeit, Verträglichkeit bzw. Sicherheit von chemischen Verbindungen in Kontakt mit Pflanzen, Pilze und Tiere erfolgen. Die chemische/n Verbindung/en kann/können als Einzelstoff oder Vielstoffgemisch getestet werden.

Beispielhaft können Nahrungsinhaltsstoffe in Lebensmitteln, Fertiggerichten, Futtermittel oder Arzneimittelstoffe für Menschen oder Wirbeltiere in pharmazeutischen Formulierungen auf Änderungen durch Verdau oder Metabolisierung und zugleich potentielle Wirkungen untersucht werden. Dies findet Anwendung im ernährungswissenschaftlichen, toxikologischen und medizinischen Bereich sowie im Life Science Bereich. Typische Einsatzgebiete für das Verfahren sind Analytik und Bewertung von Lebens- und Futtermitteln, einschließlich Functional Food, Health Food, Novel Food, Nutraceuticals, Nahrungs- und Futterergänzungsmittel, Botanicals, Hilfsstoffe, Aromen, Zusatzstoffe, weiterhin Analytik von Arzneimitteln, die den Verdauungstrakt passieren und/oder metabolisiert werden, Kosmetika oder Bedarfsgegenstände, die in den Mundraum gelangen dürfen sowie pharmazeutische oder kosmetische Formulierungen und bei der Risikobewertung bzw. Beurteilung von chemischen Stoffen hinsichtlich Verträglichkeit auf Menschen und Wirbeltieren, wie Fischen, Amphibien, Reptilien, Vögel und Säugetieren, oder auch Pflanzenzellen.

Die miniaturisierte Form auf einem gemeinsamen Träger ermöglicht verschiedene Analysen parallel automatisiert durchzuführen und auszuwerten.

Das Verfahren zur Analyse mindestens einer chemischen Verbindung einer Probe auf einem Träger nach Durchführung mindestens eines Metabolisierungsvorganges auf demselben Träger, umfasst die folgenden Schritte:
I) Auftragen der zu analysierenden Probe umfassend mind. eine chemische Verbindung auf einem Träger in einem Auftragebereich des Trägers
II) Inkontaktbringen des Trägers aus Schritt I) mit mindestens einem für den Metabolisierungsvorgang typischen Enzym, Enzymsystem und/oder Zellsystem, so dass der Metabolisierungsvorgang stattfinden kann und Metabolisierungsprodukte entstehen können
III) Chromatografische Trennung der auf demselben Träger befindlichen mindestens einen chemischen Verbindung und/oder der Metabolisierungsprodukte aus Schritt II) entlang des Trägers in einem Trennbereich, sodass sich Trennprodukte im Trennbereich befinden
IV) Detektion der Trennprodukte aus Schritt III) mittels opto-mechanischer, spektraler oder digitaler Verfahren und/oder mittels Aufbringung mindestens eines mikrochemischen Detektionsreagenzes und/oder mittels eines Assays auf dem Trennbereich des Trägers bzw. auf bestimmte Trägerbereiche
I) Auswertung der detektierten Trennprodukte aus Schritt IV)
dadurch gekennzeichnet dass
der Metabolisierungsvorgang simuliert wird durch Umsetzung mit mindestens einem für den Metabolisierungsvorgang typischen Enzym und/oder mindestens einem für den Metabolisierungsvorgang typischen Zellsystem und
dass das mindestens eine für den Metabolisierungsvorgang typische Enzym aus der Gruppe der Hydrolasen, Isomerasen, Lyasen, Ligasen, Transferasen und Oxidoreduktasen ausgewählt ist, wobei der Metabolisierungsvorgang ein Stoffwechselvorgang aus dem Mikrobiom-System oder Verdauungstrakt aus Mund-, Magen- und Darmbereich bzw. Gastrointestinalbereich (GIT) von Wirbeltieren oder ein Stoffwechselvorgang aus der Leber ist.

Der Träger umfasst Adsorbentien, die Stoffe oder gelöste Substanzen physico-chemisch binden, also adsorbieren. Es handelt sich dabei um Materialien wie z.B. Siliciumdioxid bevorzugt Kieselgel (auch Kieselsäuregel, Kieselgur oder Silikagel, silica gel genannt) oder modifizierte Kieselgele oder Polymere oder Hybridmaterialien oder ein zweiphasiges Trennmaterial. Der Träger weist eine flächige Form und zusammenhängende Konsistenz auf, zur Durchführung einer chromatografischen Trennung, um Stoffe und Stoffgemische aufzutrennen, insbesondere in einer DC. Bevorzugt wird ein leistungsstarkes Trennmaterial verwendet, z.B. HPTLC-Platte. Der Träger ist real oder imaginär in einen Aufgabebereich und einen Trennbereich aufgeteilt. Im Aufgabebereich wird die zu analysierende Probe aufgebracht. Im Trennbereich findet eine chromatografische Trennung statt. Proben bzw. Probenanteile können auch auf einen zweiten oder weitere Träger übertragen werden. Die Träger können flächig auf einem Panel angeordnet und miteinander verbunden sein.

Die zu analysierende Probe weist mindestens eine einzelne chemische Verbindung oder ein Gemisch von chemischen Verbindungen auf. Dabei umfasst die zu analysierende Probe Lebensmittel, Futtermittel, Functional Food, Health Food, Novel Food, Nutraceuticals, Nahrungs- und Futterergänzungsmittel, Botanicals, Hilfsstoffe, Aromen oder Zusatzstoffe für die Lebens- oder Futtermittel. Alternativ umfasst die zu analysierende Probe ein Arzneimittel oder Kosmetikum oder eine entsprechende Formulierung, die im menschlichen oder tierischen Körper metabolisiert werden, z.B. in den Mundraum gelangen und/oder den Verdauungstrakt passieren. Alternativ ist sie eine Umweltprobe, Pflanze, Pilz, Tier oder eine chemische Verbindung, die hinsichtlich Ihres Risikos bewertet werden muss (z.B. Migrate aus Lebensmittelverpackungen oder Kontaminanten oder Rückstände oder Verunreinigungen in Produkten). Zum Beispiel ist sie eine flüssige oder verflüssigte Probe.

Der mindestens eine Metabolisierungsvorgang ist ein Vorgang aus Stoffwechselvorgängen von Pflanzen, Pilzen und Tieren, darunter z.B. ein Vorgang aus dem Wirbeltier-Verdauungstrakt, also Mund-, Magen- und Darmbereich, sowie aus dem Gastrointestinalbereich (GIT) oder Mikrobiom. Der Metabolisierungsvorgang wird simuliert durch Umsetzung mit mindestens einem für den Metabolisierungsvorgang typischen Enzym, Enzymsystems und/oder mindestens einem für den Metabolisierungsvorgang typischen Zellsystem. Das Zellsystem umfasst ein tierisches oder pflanzliches Zellsystem, umfassend Suspensionszellen sowie adhärente Zellen ausgewählt beispielsweise aus der Gruppe der Bakterien, Hefen, Pilze, Algen und Humanzellen. Humanzellen sind beispielsweise Blutzellen, Knochenzellen, Tumorzellen oder Darmzellen.

Das mindestens eine Enzym ist z.B. ein für einen Metabolisierungsvorgang typisches Enzym des menschlichen oder tierischen Verdauungstraktes z.B. aus der Gruppe der Hydrolasen, Isomerasen, Lyasen, Ligasen, Transferasen und Oxidoreduktasen. Alternativ stammt das Enzym aus einem isolierten oder simulierten Mikrobiom.

Beispielhaft repräsentiert das mindestens eine Enzym die Vorgänge in der orale Phase im Mundbereich oder der gastrischen Phase im Magenbereich oder der intestinalen Phase im Darmbereich oder relevanter nachgeschalteter Stoffwechselvorgänge (z.B. Metabolisierung in der Leber) von Menschen oder Tieren.

Das mindestens eine Zellsystem ist ausgewählt aus der Gruppe der Pflanzen, Pilze oder Tiere. Ein besonders geeignetes Zellsystem umfasst Suspensionszellen, z.B. Hefe- (z.B. *Saccharomyces cerevisiae*), Bakterien-, Pilz-, Blut- sowie Algenzellen. Mit diesem Zellsystem wird auch die biologische Wirkung der auf dem Träger getrennten chemischen Verbindungen auf adhärente Zellen erfasst. Geeignete humane adhärente Zellen sind z.B. Osteoblasten, Leber- oder Tumorzellen wie HeLa-Zellen. Dazu werden die Zellen auf dem Träger aufgegeben und das Wachstum detektiert.

Mit diesem Zellsystem ist auch eine wirkungsbezogene Detektion möglich. Die Handhabung zur wirkungsbezogenen Detektion erfolgt nach gängigen Methoden aus dem Stand der Technik, die der Fachmann kennt. Pflanzen-, Tier- und Pilzzellen können ausgewählt werden. Für die Simulation eines Vorganges aus dem Verdauungstrakt eines Wirbeltieres, werden typische Zellsysteme oder Enzyme aus Wirbeltieren z.B. Fische, Amphibien, Reptilien, Vögel und Säugetieren, wie Menschen, Hund und Katzen eingesetzt.

Durch den Metabolisierungsvorgang auf dem Träger können ein oder mehrere Metabolisierungsprodukte auf dem Träger entstehen.

In einer Ausführungsform erfolgt der Metabolisierungsvorgang vor der chromatografischen Trennung mit der mindestens einen chemischen Verbindung der zu analysierenden Probe statt. In einer weiteren Ausführungsform findet der Metabolisierungsvorgang nach der chromatografischen Trennung der mindestens einen chemischen Verbindung der zu analysierenden Probe statt. Als weitere Sonderform kann die Trennung entfallen (bei einem reinen Einzelstoff ist eine Trennung z.B. nicht zwingend nötig).

Zum Beispiel wird erst nach der Trennung der aufgegebenen Probe das mind. eine Enzym, Enzymsystem oder Zellsystem aufgebracht. Die Auswertung erfolgt dann z.B. über die erst möglich gewordene Detektierbarkeit (ohne enzymatische Umsetzung ist sie noch nicht detektierbar) oder einen Abgleich (z.B. anhand der Zonenintensität) mit einer analogen Durchführung einer Vergleichsprobe ohne Enzym-/Zellsystemaufbringung. Mitgeführte Kontrollproben (positive/negative Proben) können die korrekte Durchführung belegen.

In einer Ausführungsform erfolgt der Metabolisierungsvorgang mit einem Enzym. Alternativ werden mehrere Enzyme oder Enzymmischungen eingesetzt. Die Enzyme werden als Mischung gleichzeitig eingesetzt oder nacheinander zugegeben. Die Zugabe nacheinander ist vorteilhaft, wenn die verwendeten Enzyme unterschiedliche Umgebungsbedingungen z.B. Temperatur benötigen, um aktiv zu sein. Der Träger kann dann erst den unterschiedlichen Umgebungsbedingungen ausgesetzt werden, bevor das Enzym zugesetzt wird. Dadurch ist es möglich, unterschiedliche Enzymtätigkeiten schrittweise zu steuern. Alternativ ist es möglich, die Enzyme nacheinander auf derselben Stelle des Trägers aufzubringen oder an verschiedenen Stellen des Trägers. Dies ist beispielsweise, wenn eine zu analysierende Probe mit unterschiedlichen Enzymen oder Enzymgemischen nacheinander reagieren soll. Wenn eine zu analysierende Probe in verschiedenen Parallelansätzen mit unterschiedlichen Enzymen oder Enzymgemischen reagiert, ist es möglich die Um- und Abbauprodukte der ersten enzymatischen Umsetzung anschließend mindestens einer weiteren enzymatischen Umsetzung auszusetzen.

Beispielsweise kann man zuerst mit aufgebrachten humanen Speichelenzymen den Mundbereich simulieren und die dort erzeugten Um- und Abbauprodukte in den Bereich der nächsten Umsetzung transportieren, die den Magenbereich mit einem aufgebrachten Pankreas-Enzymgemisch simuliert. Die Umgebungsbedingungen wie pH, Temperatur oder Zusätze wie z.B. Gallensalze sind jeweils entsprechend optimal angepasst.

In einer Ausführungsform erfolgt der Metabolisierungsvorgang im Aufgabereich. Dazu wird das mindestens eine Enzym und/oder mindestens eine Zellsystem auf dem Träger aufgebracht. Alternativ weist der Träger das mindestens eine Enzym und/oder mindestens eine Zellsystem bereits auf, bevor die zu analysierende Probe aufgebracht wird.

Die chromatografische Trennung erfolgt nach einer typischen Methode aus dem Stand der Technik. Die chromatografische Trennung umfasst planar-chromatographische Trennungen/Techniken. Bevorzugt wird dazu ein Träger mit einem leistungsstarken Trennmaterial verwendet, z.B. eine HPTLC-Platte. Die getrennten Stoffe erscheinen als Trennprodukte in einem HPTLC-Chromatogramm, das in der bildlichen Ausführungsform einfach zu erfassen ist. Die Darstellung kann aber auch als Densitogramm in Peakform erfolgen oder als 2D- bzw. 3D-Darstellung. Die Handhabung erfolgt nach gängigen Methoden und Protokollen aus dem Stand der Technik, die der Fachmann kennt.

Die Detektion der Trennprodukte erfolgt bevorzugt mittels opto-mechanischer, spektraler oder digitaler Verfahren z.B. Densitometrie, (hyper)spektrale Messysteme und digitale Bildauswerte-Systeme sowie mittels Aufbringung mindestens eines mikrochemischen Detektionsreagenzes oder eines chemischen, biochemischen oder biologischen Assays auf dem bzw. entlang des Trägers (wahlweise auch nur auf ausgewählten Bereichen des Trägers). Das Aufbringen von biologischen Assays umfasst auch das Aufbringen von adhärenten Zellen bzw. adhärenten Zellsystemen.

Detektionsreagenzien sind z.B. das 2-Naphthol-Reagenz zum Nachweis von Stärke, Ninhydrin-Reagenz zum Nachweis von Casein, Primulin-Reagenz zum Nachweis von Fetten/Ölen. Der Fachmann kennt zahlreiche weitere Derivatisierungsreagenzien zum spezifischen, selektiven und universellen Nachweis von Verbindungen. Diese werden alternativ eingesetzt, wobei die Handhabung zur Detektion nach gängigen Methoden aus dem Stand der Technik erfolgt, die der Fachmann kennt.

In einer alternativen Ausführungsform wird zum Vergleich der detektierten Trennprodukte mindestens eine weitere Probe, Vergleichsprobe, Kontrollprobe oder ein Referenzstandard mitgeführt. Die Probe kann eine negative oder positive Probe sein, wobei eine Vergleichsprobe eine bekannte oder unbekannte Probe ist, mit gleicher, ähnlicher oder ganz anderer Zusammensetzung. Die Kontrollprobe wird z.B. verwendet, um das Funktionieren des Verfahrens zu überprüfen, der Referenzstandard weist eine quantitative und/oder qualitative definierte Zusammensetzung auf. Zur beurteilenden Auswertung werden weitere Bezugssysteme herangezogen, wie z.B. Profiling/Typisierung, Fingerprinting/Fingerabdruck, Pattern recognition/Mustererkennung.

Der Vorteil des erfindungsgemäßen Verfahrens ist die Miniaturisierung und Kombination von mehreren Schritten (Umsetzung/Trennung/Detektion/Assay) auf einem einzigen Träger. Ein Konzentrationsschritt oder Umlösen in ein geeignetes Lösungsmittel ist meist nicht erforderlich. Das Verfahren ist hinsichtlich Kosten und Zeit effizient. Es können hohe Probenvolumina aufgebracht werden (z.B. bis zu 1 mL), was die Nachweisstärke verbessert. Ein weiterer Vorteil ist die erhältliche wirkungsbezogene Information, wenn wirkungsbezogene Assays zur Detektion eingesetzt werden.

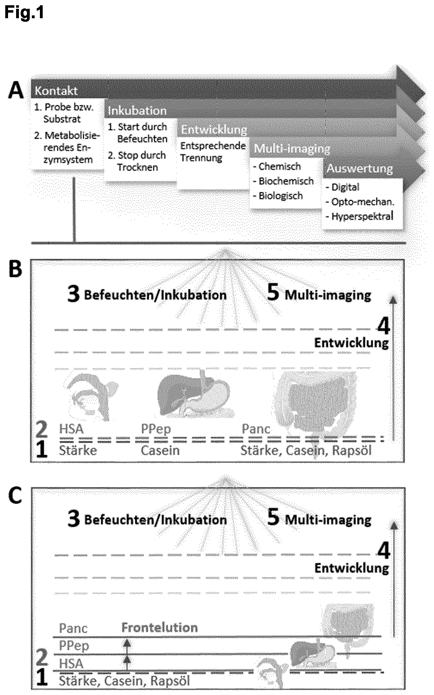

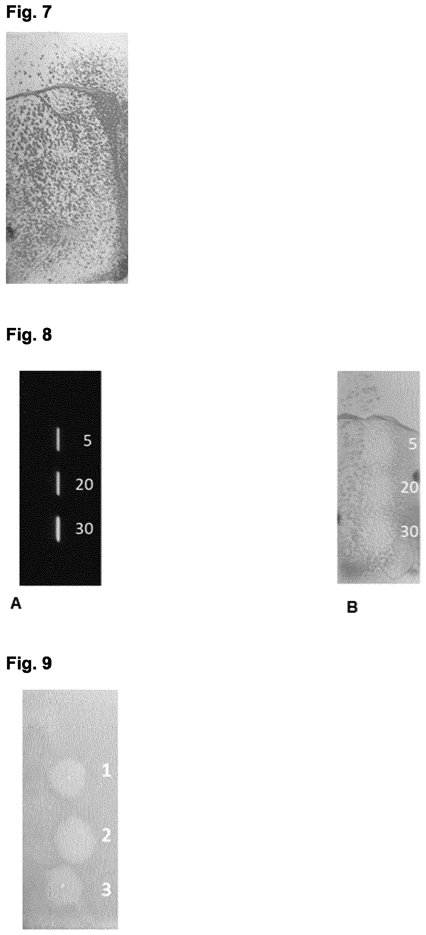

Beispielhaft werden hier Ergebnisse mit mindestens einem Enzym gezeigt aus z.B. Mund, Magen, Darm und Leber (Fig. 1-6). Die Anwendung ist aber auch auf andere Enzyme und andere Metabolisierungsvorgänge übertragbar. Beispielhaft werden hier auch Ergebnisse mit adhärenten Zellen (Fig. 7-10) gezeigt.

Beispielhaft wird als zu analysierende Probe Milcheiweiß (Casein), Kartoffelstärke, Rapsöl und Gelbwurz gezeigt. Die Anwendung ist aber auch auf weitere Proben aus Lebensmittelsystemen, Pflanzenextrakten, Botanicals, Kosmetika, Bedarfsgegenständen, Umweltproben, Migrationsstudien, Toxizitätsstudien etc. übertragbar.

Das Verfahren kann schnell und einfach auch automatisiert durchgeführt werden.

### Ausführungsbeispiele

Figur 1 zeigt einen Verfahrensablauf einer Ausführungsform. Die Schritte werden je nach Bedarf alle auf einem einzigen Träger in miniaturisierter Form ausgeführt. Dargestellt sind Beispiele für optionale Arbeitsablaufschritte (A) eines miniaturisierten *in situ* Verdaus durch enzymatische Umsetzung auf einem einzigen Träger sowie schematische Reihenfolge zweier Ausführungsbeispiele (B und C).

### Ausführungsbeispiele 1 bis 5: Lebensmittelsysteme

Die Ausführungsbeispiele zeigen verschiedene Metabolisierungsvorgänge auf einem Träger *in situ.* Der Metabolisierungsvorgang stammt aus der oralen, der gastrischen und der intestinalen Phase eines Wirbeltieres hier eines Menschen.

Es erfolgt eine enzymatische Umsetzung wurde durch in Kontakt bringen von Lebensmittelproben (Substrat) und Verdauungsflüssigkeit bzw. metabolisierende Systeme auf dem Träger. Als Substrate wurden Stärke für die orale und intestinale Phase, Casein für die gastrische und intestinale Phase, Rapsöl für die intestinale Phase und Gelbwurz für die gastrische und intestinale Phase sowie Metabolisierung mit dem S9-System in der Leber gewählt.

### Lebensmittelproben (Substrat)

- Milcheiweiß (Casein)-Lösung (5-10 µg/µL): 50-100 mg Casein wurden in 95 mL Wasser (50 °C) gerührt, 1 M NaOH wurde zugegeben (pH 11), dann mit 1 M HCl auf pH 7 eingestellt, mit Wasser auf 100 mL aufgefüllt und 5 min bei 13.000 U/min zentrifugiert.
- Rapsöllösung (1 µg/µL): 100 mg Öl wurden mit n-Hexan auf 100 mL aufgefüllt.
- Kartoffelstärkelösung (1-10 µg/µL): 100-1000 mg Stärke wurden mit Wasser (150 °C) gerührt, abgekühlt, mit Wasser auf 100 mL aufgefüllt und 5 min bei 13.000 U/min zentrifugiert.
- Gelbwurz (*Curcuma longa,* 100 µg/µL): 0,5 g einer pulverisierten Kurkumaprobe wurden mit 5 mL 60% EtOH bei 1000 U/min für 30 min gerührt, für weitere 30 min im Ultraschallbad beschallt und 15 min bei 3000 U/min zentrifugiert.

### Metabolisierende Systeme

- HSA: α-Amylase vom menschlichen Speichel (170 U/mg)
- PPep: Pepsin aus der Magenschleimhaut von Schweinen (3700 U/mg)
- Panc: Pankreatin aus Schweinepankreas (8 x USP-Spezifikationen)
- S9-Mix: S9-Enzymgemisch aus der Rattenleber induziert mit einer Mischung aus β-Naphthoflavon/Phenobarbital (0,4 mg/mL) mit 0,17 mM β-NADPH, 3 mM Glucose-6-phosphat, 5 mM Magnesiumchlorid-Hexahydrat und 0,3 U/mL Glucose-6-phosphat-Dehydrogenase

Die zuvor zur enzymatischen Umsetzung (Verdau) genannten Enzym-Systeme wurden in Salzlösungen in nachfolgenden Konzentrationen gelöst. Tabelle 1 zeigt das metabolisierende System mit entsprechenden Salzkonzentrationen.

| **Verdau** | **Orale Phase** | **Gastrische Phase** | **Intestinale Phase** |
|---|---|---|---|
| Enzym | 150 U/mL HSA | 4000 U/mL PPep | 20 mM Panc |
| Salz [mmol/L] | | | |
| K⁺ | 18.8 | 7.8 | 7.6 |
| Na⁺ | 13.6 | 72.2 | 123.4 |
| H₂PO₄⁻ | 3.7 | 0.9 | 0.8 |
| HCO₃⁻, CO₃²⁻ | 13.7 | 25.5 | 85 |
| Mg²⁺ | 0.15 | 0.1 | 0.33 |
| NH₄⁺ | 0.12 | 1.0 | - |
| Cl⁻ | 19.5 | 70.2 | 55.5 |
| Ca²⁺ | 1.5 | 0.15 | 0.6 |
| pH | 7 | 3 | 7 |

Ein möglicher Ablauf ist wie folgt.
1. Auftragen: Substratlösungen wurden je als 8 mm Band nacheinander auf HPTLC-Platten Kieselgel 60 (mit oder ohne Fluoreszenzindikator F₂₅₄) aufgetragen (15 mm Seitenabstand und 10 mm Bahnabstand).
2. Enzymlösungen wurden über die Substratbanden aufgetragen, evtl. im pH angepasst (PPep mit 1 M HCl auf pH 3 eingestellt).
3. Benetzen und Inkubation: Die Platten wurden mit 2 mL 0,1 mM Salzlösung homogen befeuchtet, bei 37 °C in einer feuchten Box dem Enzym entsprechend inkubiert und danach 5 min getrocknet.
4. Entwicklung (bei unterschiedlichen mobilen Phasen nach Plattenschnitt): Nach 5 min Kammersättigung wurde die Platte bis zu einer Laufstrecke von 6 cm entwickelt mit
   (I) 2-Propanol - Ethylacetat - Wasser 3:3:1 für die Stärke-Trennung,
   (II) 1-Butanol - Essigsäure - Wasser 4:1:1 für die Casein-Trennung,
   (III) Petroleumether - Diethylether - Essigsäure 8:2:0.1 für die Rapsöl-Trennung und
   (IV) Petroleumether - Ethylacetat - Cyclohexan 5:2.2:2.8 für die Gelbwurztrennung sowie anschließend getrocknet.
5. Detektion: Das β-Naphtholreagenz wurde für die Detektion der Metabolisierungsprodukte Glucose und Maltose aus Stärke eingesetzt. Die Absorptionsmessung erfolgte bei 434 nm. Das Ninhydrinreagenz wurde für die Detektion der Metabolisierungsprodukte (Aminosäuren und Peptide) von Kasein eingesetzt. Die Absorptionsmessung erfolgte bei 520 nm. Das Primulinreagenz wurde für die Detektion der Metabolisierungsprodukte des Rapsöls eingesetzt. Die Fluoreszenzmessung erfolgte bei 366/>400 nm. Als Beispiel für eine wirkungsbezogene Detektion wurde Gelbwurz mit dem Tyrosinaseassay detektiert. Die Absorptionsmessung erfolgte als inverser Scan bei 546 nm.

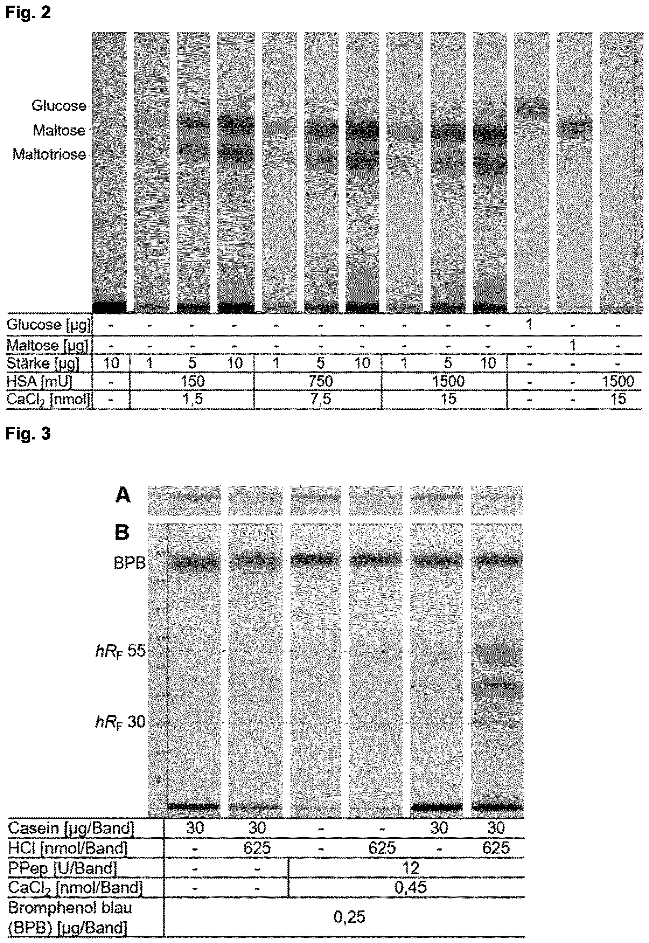

### Ausführungsbeispiel 1: Optimierungsversuch für die Mundphase (Figur 2)

- Enzymatische Umsetzung der Stärke mit verschiedenen Enzym- und Substratmengen mit einer Inkubationszeit von 2 min für die orale Phase
- Unterschiedlich verdaute Stärke entwickelt mit mobiler Phase I, derivatisiert mit 2-Naphthol-Reagenz und dokumentiert unter Weißlicht-Beleuchtung
- Zum Signalabgleich wurden Einzellösungen als Blindwert und Kontrollprobe mitgeführt
- Eruieren der optimalen Menge/Konzentration für Kartoffelstärke als Substrat, humane Speichelamylase (HSA) und Calciumclorid

Figur 2 zeigt den simulierten oralen Verdau miniaturisiert *in situ* des Adsorbens: Beispiel für die Optimierung der Metabolisierung von Kartoffelstärke.

### Ausführungsbeispiel 2: Optimierungsversuch für die Magenphase (Figur 3)

- Enzymatische Umsetzung des Casein-Milcheiweißes mit zuvor optimierten Enzym- (Pepsin, PPep) und Substratmengen bei einer Inkubationszeit von 60 min für die gastrische Verdauphase
- Verdautes Milcheiweiß entwickelt mit mobiler Phase II, derivatisiert mit Ninhydrin-Reagenz und dokumentiert unter Weißlicht-Beleuchtung
- Zum Signalabgleich wurden Einzellösungen und Kombinationen als Blindwert und Kontrollprobe mitgeführt

Figur 3 zeigt den simulierte gastrischen Verdau miniaturisiert *in situ* des Adsorbens: Beispiel für die Metabolisierung von Casein-Milcheiweiß mit zuvor optimierten Enzym- und Substratmengen zu Aminosäuren und Peptiden v. a. im Bereich *hR*_{F} 30 bis 55 (A: Zusatz von Indikator Bromphenolblau (BPB) als Kontrolle der auf pH 3 eingestellten Inkubationszonen) und als Abgleich Einzellösungen bzw. Kombinationen (B).

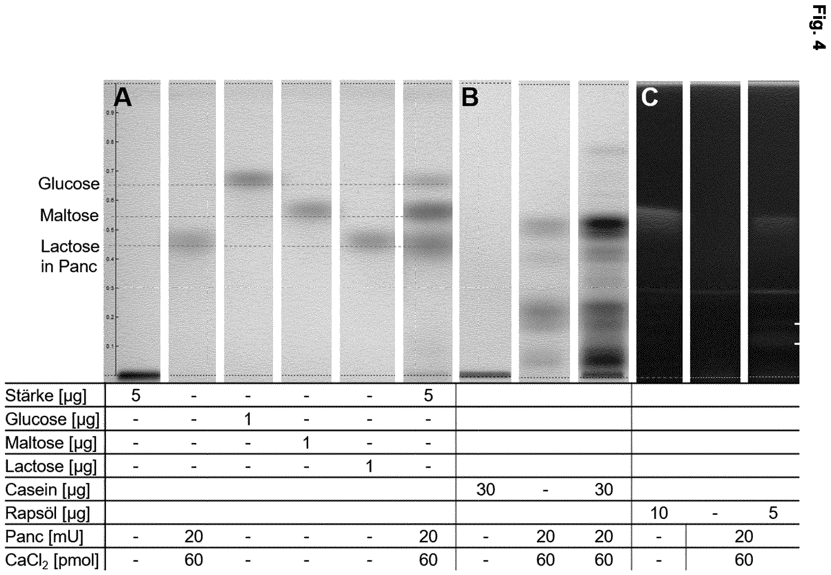

### Ausführungsbeispiel 3: Optimierungsversuch für die Darmphase (Figur 4)

- Enzymatische Umsetzung von Kartoffelstärke, Casein-Milcheiweiß und Rapsöl durch Dünndarmenzyme (Pancreatin, Panc) mit zuvor optimierten Enzym- und Substratmengen bei einer Inkubationszeit von 60 min für die intestinale Verdauphase
- Verdaute Lebensmittel entwickelt mit entsprechender mobiler Phase I bis III, derivatisiert mit 2-Naphthol- (Fig. 4A), Ninhydrin (Fig. 4B), und Primulin-Reagenz (Fig. 4C) und dokumentiert unter Weißlicht-Beleuchtung (Abb. 4A/B) sowie UV 366 nm (Fig. 4C)
- Zum Signalabgleich wurden Einzellösungen und Kombinationen als Blindwert und Kontrollprobe mitgeführt

Figur 4 zeigt den simulierten intestinalen Verdau miniaturisiert *in situ* des Adsorbens: Beispiel für die Metabolisierung von Kartoffelstärke (A), Casein-Milcheiweiß (B) und Rapsöl (C, entstandene Fettsäuren markiert) mit zuvor optimierten Mengen sowie als Abgleich Einzellösungen bzw. Kombinationen (B).

### Ausführungsbeispiel 4: Vergleich in situ versus in vitro Verdau

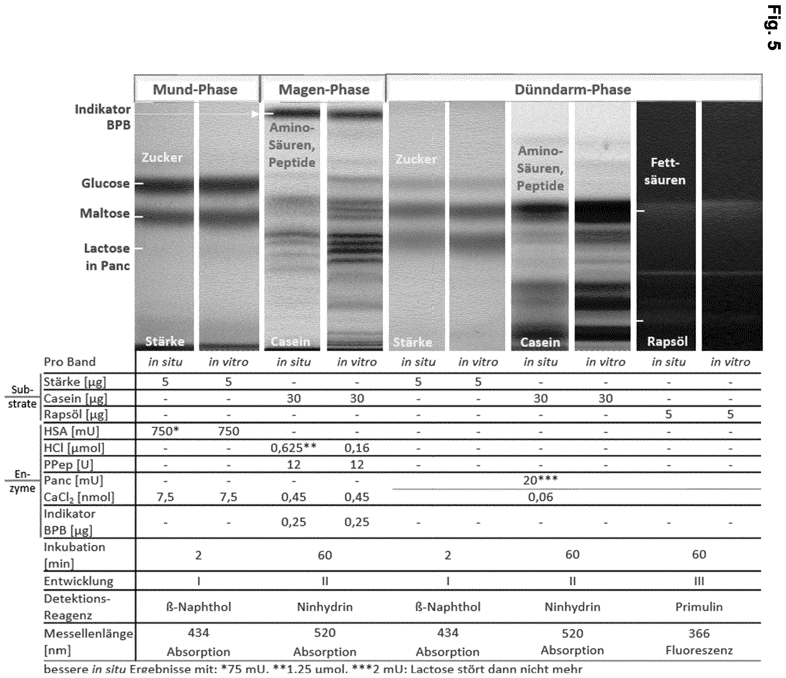

Für den direkten Vergleich von *in situ* Verdau mit *in vitro* Verdau wurden vergleichbare (z.T. nicht optimale Bedingungen für den *in situ* Verdau) Enzym- und Substratkonzentrationen verwendet und beide Verdau-Systeme auf der gleichen Platte gezeigt (Figur 5). Enzym- und Substrat-Lösungen wurden für den *in situ* Verdau nacheinander aufgebracht. Während diese Platte befeuchtet wurde (Start des *in situ* Verdaus), wurden zeitgleich die Lösungen für den *in vitro* Verdau gemischt (alle 10 min erneut gemischt und nach der Inkubation zentrifugiert).

Beide Verdau-Systeme wurden bei 37 °C für die gleiche Dauer inkubiert (*in situ* auf der Platte und *in vitro* im Glasgefäß). Auf die getrocknete *in situ* Verdau-Platte wurden die *in vitro* Verdaulösungen aufgetragen. Entwicklung und Derivatisierung wurden wie erwähnt durchgeführt.

Figur 5 zeigt den direkten Vergleich von *in situ* Verdau mit *in vitro* Verdau unter der Verwendung von vergleichbaren Enzym- und Substratkonzentrationen; beim *in situ* Verdau finden Verdau, Trennung und Detektion auf derselben Trägerplatte statt.

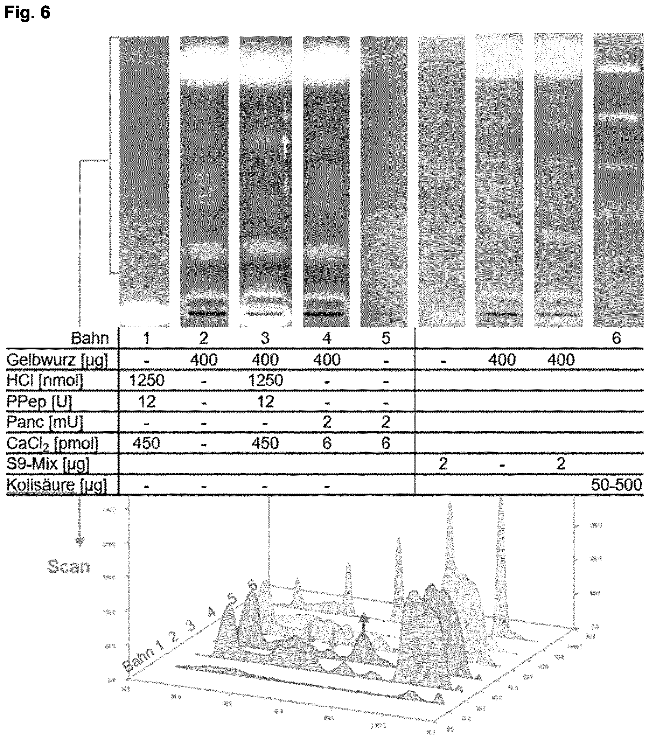

### Ausführungsbeispiel 5: Anwendungsbeispiel für den simulierten Verdau von Gelbwurz in der gastrischen und intestinalen Phase und Metabolisierung mit dem S9-System der Leber (Figur 6)

- Enzymatische Umsetzung von Gelbwurzextrakt durch Magen- (PPep) und Dünndarmenzyme (Panc) sowie S9-Gemisch (S9-Mix)
- Gelbwurzextrakt entwickelt mit mobiler Phase IV, detektiert nach dem wirkungsbezogenen Tyrosinase-Hemmassay (helle Zonen zeigen Tyrosinase hemmende Substanzen) und dokumentiert unter Weißlicht-Beleuchtung
- Zum Signalabgleich wurden Einzellösungen bzw. Kombinationen als Blindwert und Kontrollprobe mitgeführt

Figur 6 zeigt die Anwendung des simulierten Verdaus bzw. der Metabolisierung in der Leber miniaturisiert *in situ* des Adsorbens am Beispiel von Gelbwurzextrakt (Verdauungsenzyme der gastrischen und intestinalen Phase sowie S9-Mix-System in der Leber zum Vergleich) in Kombination mit der Wirkungsbezogenen Detektion mit dem Tyrosinaseassay und dokumentiert im sichtbaren Bereich (helle Zonen zeigen Tyrosinase-hemmende Substanzen; Positivkontrollstandard Kojisäure als Kalibrationsbandreihe auf Bahn 6)

### Ausführungsbeispiel 6 Wirkungsbezogene Detektion mit adhärenten Zellen

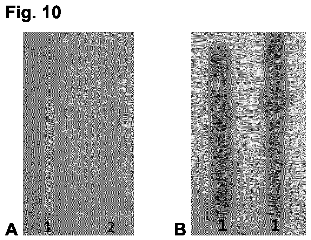

In Ausführungsbeispiel 6 werden adhärente Zellen z.B. HeLa-Zellen verwendet. Diese werden auf dem Träger erstmalig zum Wachsen gebracht und auf dem Träger vital bzw. durch Substanzen beeinflusst detektiert (nachfolgend mit 4 Testergebnissen belegt). HeLa-Zellen stammen vom Menschen (*Homo sapiens*) und gehören zum Zelltyp Zervixkarzinom. Ihre Herkunft entstammt 1951 aus epitheloidem Zervixkarzinom einer 31-jährigen farbigen Frau. Die Diagnose wurde später in Adenokarzinom geändert. Es handelt sich um die erste aneuploide, kontinuierlich kultivierte menschliche Zelllinie mit epithelial-ähnlichen Zellen, die in Monolayern wachsen.
1) Es erfolgt eine flächige Zellauftragung mit adhärenten HeLa-Zellen auf dem Träger, dann eine Inkubationsdauer von 18 Stunden. Die Zellviabiliät wurde mit MTT getestet. Die aufgebrachten und inkubierten Zellen überleben auf dem Träger und sind als dunkle Punkte auf dem Träger zu erkennen (Figur 7).
   Der MTT-Test ist ein Test zur Bestimmung der metabolischen Aktivität von Zellen. Da diese unter bestimmten Bedingungen stark mit der Zellviabilität korreliert, wird der MTT-Test häufig genutzt, um diese zu evaluieren. Dazu werden Zellen mit dem namensgebenden gelben Tetrazoliumsalz behandelt, um ihre Lebensfähigkeit beziehungsweise den Anteil lebender Zellen im Vergleich zu einer Kontrollprobe von Zellen zu messen. Dies ist beispielsweise nach Testung einer giftigen Substanz notwendig. Der Nachweis der Zellvitalität mittels MTT-Test beruht auf der Reduktion des gelben, wasserlöslichen Farbstoffs 3-(4,5-Dimethylthiazol-2-yl)-2,5-diphenyltet-razoliumbromid (MTT) in ein blau-violettes, wasserunlösliches Formazan. Das Verfahren wurde nach Standardangaben durchgeführt und ist dem Fachmann bekannt.
2) Es erfolgt die bandförmige Applikation von Resveratrol auf dem Träger. Resveratrol ist eine organische Verbindung mit der Summenformel C₁₄H₁₂O₃ aus der Gruppe der Polyphenole. Es zählt zu den Phytoalexinen mit antioxidativen Eigenschaften und ist auch in Diskussion als Verbindung, die gegen diverse Krebszellen wirken könnte. Figur 8 A zeigt die aufgebrachten verschiedenen Konzentrationen von Resveratrol (5-30 µg/Band) als helles, blau fluoreszierendes Band durch Fluoreszenzdetektion bei 366 nm. Nun erfolgt zudem die flächige Auftragung von adhärenten HeLa-Zellen auf den Träger. Die Inkubationsdauer beträgt 24 Stunden und die Zellviabiliät wird mit MTT getestet. Figur 8 B zeigt das Ergebnis nach HeLa-Bioassay im Weißlicht und verschiedenen Konzentrationen von Resveratrol. Es ist eine deutliche Abnahme der Krebszellvitalität durch das Ausbleiben der Formazan-Farbstoff-Bildung in den drei hellen Resveratrol-Zonen zu erkennen (durch die lange Inkubationszeit sind die anfänglich scharfen Banden diffundiert und breiter geworden). Die Zellen außerhalb sind hingegen noch nach 24 Stunden vital.
3) Es erfolgt eine tropfenweise Applikation von Methanol. Die Zellauftragung erfolgt flächig und die Inkubationsdauer beträgt 18 Stunden. Die Zellviabiliät wird getestet mit Resazurin. Resazurin ist ein Farbstoff, der als Redoxindikator und pH-Indikator eingesetzt wird. Dabei kann Resazurin sowohl Redoxreaktionen als auch pH-Wert-Änderungen anzeigen. Es wird als Reagenz auf Hyposulfite sowie zur Messung der Zellviabilität und der Zytotoxizität von Stoffen verwendet. Figur 9 zeigt das Ergebnis der adhärenten HeLa-Zellen unter dem Einfluss von verschiedenen Methanolkonzentrationen (1-3 µL Tropfen; nach dem Bioassay detektiert im Weißlicht). Der Nachweis der Zellviabilität mittels Resazurin führte zu einem orange-rot fluoreszierenden bzw. helleren Hintergrund - dort im Hintergrund sind die Zellen vital. Die aufgetropften runden, dunkleren (violetten) Methanol-Flächen zeigen, dass hier kein Metabolismus der HeLa-Zellen mehr stattfindet (Abtötung der Krebszellen durch das Zellgift Methanol).
4) Es erfolgt ein bandförmiges Aufpipettieren der HeLa-Zellen und ein Nachweis der Zellviabiliät auf dem Träger mit Resazurin (Inkubationsdauer 15 Stunden) sowie MTT (Inkubationsdauer 4,5 Stunden). Figur 10 A zeigt als langes Band aufpipettierte HeLa-Zellen, die auf dem Träger wachsen (1, vital als helles bzw. orange-rot fluoreszierendes Band). Als Kontrollprobe wird das bloße Medium als Negativkontrolle (2, enthält keine Zellen; trägt nicht zum hellen Signal bei) aufgetragen. Der Nachweis der Zellviabilität mittels Resazurin führte zu einer hellen (orange-roten) Bandfläche, dort sind die Zellen vital (auf dunklem bzw. violetten Hintergrund). Die Auftragung des Mediums zeigt, dass das Medium keinen Einfluss auf die helle bzw. orange-rote Resazurin-Färbung hat. Figur 10 B zeigt zur Wiederholung zwei Mal bandförmig aufpipettierte HeLa-Zellen (1) und die Auswertung durch MTT im Weißlicht. Die Färbung mit MTT führt zu zwei dunkleren bandenförmigen Zellflächen. Hier sind die Zellen ebenfalls vital und die Farbentwicklung belegt die Vitalität der adhäsiven Zellen auf dem Träger (auf hellem Hintergrund, denn dort findet kein Metabolismus bzw. keine Färbung statt, da dort keine Zellen sind).

### Kultivierung der HeLa-Zellen

1. Die HeLa-Zellen wurden in 75-cm²-Kulturflaschen (37°C, 5% CO₂, 100% Luftfeuchtigkeit) kultiviert, bis sie zu etwa 90% konfluent waren. Für den Bioassay wurden die Zellen trypsiniert, in einen 15 mL Falcon transferiert und bei 2000 x g zentrifugiert. Anschließend wurden die Zellen resuspendiert, gezählt und auf die erforderliche Zellzahl eingestellt.
2. Bevor die HeLa-Zellen auf die RP-18 W-Platte bzw. nach der chromatographischen Trennung auf das entsprechende Chromatogramm pipettiert werden konnten, musste die Platte in PBS-Lösung (2-fach konzentriert; 19,1 g/L) getaucht werden, so dass die Platte vollständig befeuchtet wurde. Die Platte wurde in eine vorbereitete Inkubationskammer gelegt. Es wurde wenige Minuten gewartet, bis die überschüssige Feuchtigkeit (glänzende Oberfläche) verschwunden war.
3. Die zu testenden Wirkzonen und darauf aufpipettierten Hela-Zellen müssen von allen Seiten gleichmäßig (gleicher Abstand zu weiterer Feuchtigkeitsquelle) feucht gehalten werden. In diesem Beispiel wurden aufgrund des oberen freien Plattenbereichs (größere Abdrift der Feuchtigkeit nach oben), 200 µL einer 10%i-gen Glukoselösung oder doppelt-konzentrierten PBS-Lösung auf das obere Ende der Platte pipettiert. Diese Feuchtigkeitszone diente als Feuchtigkeitsreservoir für den oberen Bereich.
4. Die Zellsuspension (200 µL) wurde zügig der Länge nach auf die Platte aufpipettiert und die Inkubationskammer anschließend mit einer Glasplatte abgedeckt. Die Inkubationskammer wurde 10 Minuten bei Raumtemperatur stehen gelassen, damit die Zellen sedimentieren konnten und dann für 2 - 24 Stunden in den CO₂-Brutschrank gestellt. Nach der erforderlichen Inkubationszeit wurde die Platte aus der Inkubationskammer entfernt und die Glukoselösung mit einem Zellulosestreifen entfernt, bevor die Platte vertikal auf ein Zellstofftuch gestellt wurde, damit die Kulturlösung ablaufen konnte. Für die Zellviabilitätstests wurden die Platten nach dem Entfernen der Kulturflüssigkeit nass in MTT (2 mg/mL PBS) oder Resazurin (0,4 mg/mL PBS) getaucht. Die Platten wurden wieder in die Inkubationskammer zurückgelegt und für weitere 2 - 20 Stunden im CO₂-Brutschrank inkubiert.

### Abbildungslegenden und Bezugszeichenliste

Fig. 1 Beispiele für optionale Arbeitsablaufschritte (A) eines miniaturisierten *in situ* Verdaus auf einem einzigen Träger sowie schematische Reihenfolge zweier Ausführungsbeispiele (B und C)
Fig. 2 Simulierter oraler Verdau miniaturisiert *in situ* des Adsorbens: Beispiel für die Optimierung der Metabolisierung von Kartoffelstärke
Fig. 3 Simulierter gastrischer Verdau miniaturisiert *in situ* des Adsorbens: Beispiel für die Metabolisierung von Casein-Milcheiweiß mit zuvor optimierten Enzym- und Substratmengen zu Aminosäuren und Peptiden v. a. im Bereich *hR*_{F} 30 bis 55 (A: Zusatz von Indikator Bromphenolblau (BPB) als Kontrolle der auf pH 3 eingestellten Inkubationszonen) und als Abgleich Einzellösungen bzw. Kombinationen (B)
Fig. 4 Simulierter intestinaler Verdau miniaturisiert *in situ* des Adsorbens: Beispiel für die Metabolisierung von Kartoffelstärke (A), Casein-Milcheiweiß (B) und Rapsöl (C), entstandene Fettsäuren markiert) mit zuvor optimierten Mengen sowie als Abgleich Einzellösungen bzw. Kombinationen (B)
Fig. 5 Direkter Vergleich von *in situ* Verdau mit *in vitro* Verdau unter der Verwendung von vergleichbaren Enzym- und Substratkonzentrationen; beim *in situ* Verdau finden Verdau, Trennung und Detektion auf derselben Trägerplatte statt
Fig. 6 Anwendung des simulierten Verdaus bzw. der Metabolisierung in der Leber miniaturisiert *in situ* des Adsorbens am Beispiel von Gelbwurzextrakt (Verdauungsenzyme der gastrischen und intestinalen Phase sowie S9-Mix-System in der Leber zum Vergleich) in Kombination mit der Wirkungsbezogenen Detektion mit dem Tyrosinaseassay und dokumentiert im sichtbaren Bereich (helle Zonen zeigen Tyrosinase hemmende Substanzen; Positivkontrollstandard Kojisäure als Kalibrationsband auf Bahn 6)
Fig. 7 Inhomogene Auftragung von adhärenten Zellen nach 18 Stunden Inkubation vital und als dunkle Punkte auf dem Träger
Fig. 8 Wirkungsbezogene Detektion mit adhärenten Zellen. A) zeigt das Ergebnis der Fluoreszenzmessung bei 366 nm und verschiedenen Konzentrationen von Resveratrol. B) zeigt das Ergebnis nach Bioassay im Weißlicht und verschiedenen Konzentrationen von Resveratrol (5, 20 und 30 µg/Band).
Fig. 9 Verfahrensergebnis der adhärenten Zellen bei verschiedenen Methanolkonzentrationen (1, 2 und 3 µL-Tropfen) nach einem Bioassay mit Weißlicht. Der Nachweis der Zellviabilität mittels Resazurin führte zu einem helleren Hintergrund.
Fig. 10 Verfahrensergebnis nach bandenförmigem Aufpipettieren der HeLa-Zellen. Die Zellviabiliät wurde getestet mit Resazurin (Inkubationsdauer 15 Stunden) und MTT (Inkubationsdauer 4,5 Stunden). (A) Aufpipettierte Zellen (1) im Gegensatz zu Medium (2). (B) aufpipettierte Zellen (1) im Gegensatz zu Medium nach Zugabe von Aufgabe von Resazurin (366 nm) und Auswertung durch MTT im Weißlicht.

## Patentansprüche

1. Verfahren zur Analyse mindestens einer chemischen Verbindung einer Probe auf einem Träger nach Durchführung mindestens eines Metabolisierungsvorganges auf demselben Träger, umfassend die folgenden Schritte:
I) Auftragen der zu analysierenden Probe umfassend mind. eine chemische Verbindung auf einem Träger in einem Auftragebereich des Trägers
II) Inkontaktbringen des Trägers aus Schritt I) mit mindestens einem für den Metabolisierungsvorgang typischen Enzym, Enzymsystem und/oder Zellsystem, so dass der Metabolisierungsvorgang stattfinden kann und Metabolisierungsprodukte entstehen können
III) Chromatografische Trennung der auf demselben Träger befindlichen mindestens einen chemischen Verbindung und/oder der Metabolisierungsprodukte aus Schritt II) entlang des Trägers in einem Trennbereich, sodass sich Trennprodukte im Trennbereich befinden
IV) Detektion der Trennprodukte aus Schritt III) mittels opto-mechanischer, spektraler oder digitaler Verfahren und/oder mittels Aufbringung mindestens eines mikrochemischen Detektionsreagenzes und/oder mittels eines Assays auf dem Trennbereich des Trägers bzw. auf bestimmte Trägerbereiche
V) Auswertung der detektierten Trennprodukte aus Schritt IV)
**dadurch gekennzeichnet dass**
der Metabolisierungsvorgang simuliert wird durch Umsetzung mit mindestens einem für den Metabolisierungsvorgang typischen Enzym und/oder mindestens einem für den Metabolisierungsvorgang typischen Zellsystem und
dass das mindestens eine für den Metabolisierungsvorgang typische Enzym aus der Gruppe der Hydrolasen, Isomerasen, Lyasen, Ligasen, Transferasen und Oxidoreduktasen ausgewählt ist, wobei der Metabolisierungsvorgang ein Stoffwechselvorgang aus dem Mikrobiom-System oder Verdauungstrakt aus Mund-, Magen- und Darmbereich bzw. Gastrointestinalbereich (GIT) von Wirbeltieren oder ein Stoffwechselvorgang aus der Leber ist.

2. Verfahren gemäß Anspruch 1 **dadurch gekennzeichnet, dass** der Träger Adsorbentien umfasst, die Stoffe oder gelöste Substanzen adsorbieren und ausgewählt sind aus der Gruppe Kieselgel, Kieselsäuregel, Kieselgur oder Silikagel, sowie modifiziertes Kieselgel, Polymere oder Hybridmaterialien oder mehrphasiges Trennmaterial.

3. Verfahren gemäß der Ansprüche 1 oder 2 **dadurch gekennzeichnet, dass** die zu analysierende Probe mindestens eine chemische Verbindung oder ein Lebensmittel, Futtermittel, Functional Food, Health Food, Novel Food, Nutraceutical, Nahrungs- und Futterergänzungsmittel, Pflanzenstoff, Hilfsstoff, Aromastoff, Zusatzstoff, Arzneimittel, Kosmetikum, Umweltprobe oder entsprechende Formulierungen umfasst.

4. Verfahren gemäß einem der vorangegangenen Ansprüche 1 bis 3 **dadurch gekennzeichnet, dass** das mindestens eine für den Metabolisierungsvorgang typische Zellsystem ausgewählt ist aus der Gruppe der Suspensionszellsysteme oder adhäsiven Zellsysteme.

5. Verfahren gemäß einem der vorangegangenen Ansprüche 1 bis 4 **dadurch gekennzeichnet, dass** der Organismus der Suspensionszellsysteme oder adhäsiven Zellsysteme ausgewählt ist aus der Gruppe der Pflanzen, Tiere und Pilze.

6. Verfahren gemäß einem der vorangegangenen Ansprüche 1 bis 5 **dadurch gekennzeichnet, dass** die chromatografische Trennung die Verfahren der Planar-Chromatographie umfasst.

7. Verfahren gemäß einem der vorangegangenen Ansprüche 1 bis 6 **dadurch gekennzeichnet, dass** die Detektion der Trennprodukte nach der chromatografischen Trennung mittels opto-mechanischer, spektraler oder digitaler Verfahren oder durch Aufbringung mindestens eines mikrochemischen Detektionsreagenzes oder eines chemischen, biochemischen oder biologischen Assays erfolgt.

8. Verfahren gemäß einem der vorangegangenen Ansprüche 1 bis 7 **dadurch gekennzeichnet, dass** die Detektionsreagenzien stoffspezifisch ausgewählt sind, wie z.B. 2-Naphthol-Reagenz zum Nachweis von Stärke, Ninhydrin-Reagenz zum Nachweis von Casein, Primulin-Reagenz zum Nachweis von Fetten und Ölen, oder Wirkungsdetektion zum Nachweis von Gelbwurzextrakt.

9. Verfahren gemäß einem der vorangegangenen Ansprüche 1 bis 8 **dadurch gekennzeichnet, dass** die Auswertung der detektierten Trennprodukte im Vergleich mit weiteren Proben, Vergleichsproben oder Kontrollproben oder Referenzstandards definierter Zusammensetzung auf dem Träger erfolgt.

10. Verfahren gemäß einem der vorangegangenen Ansprüche 1 bis 9 **dadurch gekennzeichnet, dass** die Auswertung der detektierten Trennprodukte mittels Profiling/Typisierung, Fingerprinting/Fingerabdruck, Pattern recognition/Mustererkennung oder ähnlichen Verfahren erfolgt.

11. Verfahren gemäß einem der vorangegangenen Ansprüche 1 bis 10 **dadurch gekennzeichnet, dass** die Auswertung der detektierten Trennprodukte mittels Multi-Imaging oder wirkungsbezogen erfolgt.

12. Verfahren gemäß Anspruch 11 **dadurch gekennzeichnet, dass** die wirkungsbezogene Auswertung der detektierten Trennprodukte durch Zugabe von adhärenten Zellen erfolgt.

## Claims

1. Method for analyzing at least one chemical compound of a sample on a support after carrying out at least one metabolization process on the same support, comprising the following steps:
I) Application of the sample to be analyzed comprising at least one chemical compound on a carrier in an application area of the carrier
II) Contacting the carrier from step I) with at least one enzyme, enzyme system and/or cell system typical for the metabolization process, so that the metabolization process can take place and metabolization products can be formed
III) Chromatographic separation of the at least one chemical compound present on the same carrier and/or the metabolization products from step II) along the carrier in a separation region, so that separation products are present in the separation region
IV) Detection of the separation products from step III) by means of opto-mechanical, spectral or digital methods and/or by applying at least one microchemical detection reagent and/or by means of an assay to the separation region of the carrier or to specific carrier regions
I) Evaluation of the detected separation products from step IV **characterized in that**
the metabolization process is simulated by reaction with at least one enzyme typical of the metabolization process and/or at least one cell system typical of the metabolization process, and
that the at least one enzyme typical for the metabolization process is selected from the group of hydrolases, isomerases, lyases, ligases, transferases and oxidoreductases, wherein the metabolization process is a metabolic process from the microbiome system or digestive tract from the mouth, stomach and intestinal region or gastrointestinal region (GIT) of vertebrates or a metabolic process from the liver.

2. Method according to claim 1, **characterized in that** the carrier comprises adsorbents which adsorb substances or dissolved substances and are selected from the group consisting of silica gel, silicic acid gel, diatomaceous earth or silica gel, as well as modified silica gel, polymers or hybrid materials or multiphase separation material.

3. Method according to claims 1 or 2, **characterized in that** the sample to be analysed comprises at least one chemical compound or a food, feed, functional food, health food, novel food, nutraceutical, food and feed supplement, plant substance, excipient, flavouring agent, additive, pharmaceutical, cosmetic, environmental sample or corresponding formulations.

4. Method according to one of the preceding claims 1 to 3, **characterized in that** the at least one cell system typical for the metabolization process is selected from the group of suspension cell systems or adhesive cell systems.

5. Method according to any of the preceding claims 1 to 4, **characterized in that** the organism of the suspension cell systems or adhesive cell systems is selected from the group consisting of plants, animals and fungi.

6. Method according to any one of the preceding claims 1 to 5, **characterized in that** the chromatographic separation comprises the processes of planar chromatography.

7. Method according to one of the preceding claims 1 to 6, **characterized in that** the detection of the separation products after the chromatographic separation is carried out by means of opto-mechanical, spectral or digital methods or by applying at least one microchemical detection reagent or a chemical, biochemical or biological assay.

8. Method according to one of the preceding claims 1 to 7, **characterized in that** the detection reagents are selected substance-specifically, such as 2-naphthol reagent for the detection of starch, ninhydrin reagent for the detection of casein, primulin reagent for the detection of fats and oils, or effect detection for the detection of turmeric extract.

9. Method according to one of the preceding claims 1 to 8, **characterized in that** the evaluation of the detected separation products is carried out in comparison with further samples, reference samples or control samples or reference standards of defined composition on the carrier.

10. Method according to one of the preceding claims 1 to 9, **characterized in that** the evaluation of the detected separation products is carried out by means of profiling/typing, fingerprinting/fingerprinting, pattern recognition or similar methods.

11. Method according to one of the preceding claims 1 to 10, **characterized in that** the evaluation of the detected separation products is carried out by means of multi-imaging or effect-based.

12. Method ccording to claim 11, **characterized in that** the effect-related evaluation of the detected separation products is carried out by adding adherent cells.

## Revendications

1. Procédé d'analyse d'au moins un composé chimique d'un échantillon sur un support après réalisation d'au moins une opération de métabolisation sur le même support, comprenant les étapes suivantes:
I) dépôt de l'échantillon à analyser comprenant au moins un composé chimique sur un support dans une zone de dépôt du support
II) Mise en contact du support de l'étape I) avec au moins une enzyme, un système enzymatique et/ou un système cellulaire typique du processus de métabolisation, de sorte que le processus de métabolisation puisse avoir lieu et que des produits de métabolisation puissent se former.
III) séparation chromatographique du au moins un composé chimique se trouvant sur le même support et/ou des produits de métabolisation de l'étape II) le long du support dans une zone de séparation, de sorte que des produits de séparation se trouvent dans la zone de séparation
IV) détection des produits de séparation de l'étape III) par des procédés opto-mécaniques, spectraux ou numériques et/ou par l'application d'au moins un réactif de détection microchimique et/ou par un dosage sur la zone de séparation du support ou sur certaines zones du support.
I) évaluation des produits de séparation détectés à l'étape IV)
**caractérisé en ce que**
le processus de métabolisation est simulé par réaction avec au moins une enzyme typique pour le processus de métabolisation et/ou au moins un système cellulaire typique pour le processus de métabolisation et
**en ce que** l'au moins une enzyme typique du processus de métabolisation est choisie dans le groupe des hydrolases, des isomérases, des lyases, des ligases, des transférases et des oxydoréductases, le processus de métabolisation étant un processus métabolique issu du système microbien ou du système digestif de la région buccale, gastrique et intestinale ou de la région gastro-intestinale (GIT) de vertébrés ou un processus métabolique issu du foie.

2. Procédé selon la revendication 1, **caractérisé en ce que** le support comprend des adsorbants qui adsorbent des substances ou des substances dissoutes et qui sont choisis dans le groupe constitué par le gel de silice, le gel d'acide silicique, la terre de diatomées ou le gel de silice, ainsi que le gel de silice modifié, des polymères ou des matériaux hybrides ou un matériau de séparation à plusieurs phases.

3. Procédé selon les revendications 1 ou 2, **caractérisé en ce que** l'échantillon à analyser comprend au moins un composé chimique ou un aliment, un aliment pour animaux, un aliment fonctionnel, un aliment de santé, un nouvel aliment, un nutraceutique, un complément alimentaire et un complément alimentaire pour animaux, une substance végétale, un excipient, un arôme, un additif, un médicament, un cosmétique, un échantillon environnemental ou des formulations correspondantes.

4. Procédé selon l'une quelconque des revendications 1 à 3 précédentes, **caractérisé en ce que** ledit au moins un système cellulaire typique du processus de métabolisation est choisi dans le groupe des systèmes cellulaires en suspension ou des systèmes cellulaires adhésifs.

5. Procédé selon l'une des revendications 1 à 4 précédentes, **caractérisé en ce que** l'organisme des systèmes de cellules en suspension ou des systèmes de cellules adhésives est choisi dans le groupe des plantes, des animaux et des champignons.

6. Procédé selon l'une quelconque des revendications 1 à 5 précédentes, **caractérisé en ce que** la séparation chromatographique comprend les procédés de chromatographie planaire.

7. Procédé selon l'une quelconque des revendications 1 à 6 précédentes, **caractérisé en ce que** la détection des produits de séparation après séparation chromatographique est effectuée par des méthodes opto-mécaniques, spectrales ou numériques ou par application d'au moins un réactif de détection microchimique ou d'un dosage chimique, biochimique ou biologique.

8. Procédé selon l'une quelconque des revendications 1 à 7 précédentes, **caractérisé en ce que** les réactifs de détection sont choisis spécifiquement pour chaque substance, comme par exemple le réactif au 2-naphtol pour la détection de l'amidon, le réactif à la ninhydrine pour la détection de la caséine, le réactif à la primuline pour la détection des graisses et des huiles, ou la détection d'action pour la détection de l'extrait de curcuma.

9. Procédé selon l'une des revendications 1 à 8 précédentes, **caractérisé en ce que** l'évaluation des produits de séparation détectés s'effectue en comparaison avec d'autres échantillons, des échantillons de comparaison ou des échantillons de contrôle ou des étalons de référence de composition définie sur le support.

10. Procédé selon l'une des revendications 1 à 9 précédentes, **caractérisé en ce que** l'évaluation des produits de séparation détectés s'effectue au moyen d'un profilage/typage, d'une empreinte digitale/fingerprinting, d'une reconnaissance de modèle/pattern recognition ou de procédés similaires.

11. Procédé selon l'une des revendications 1 à 10 précédentes, **caractérisé en ce que** l'évaluation des produits de séparation détectés s'effectue au moyen d'une imagerie multiple ou en fonction de l'effet.

12. Procédé selon la revendication 11, **caractérisé en ce que** l'évaluation en fonction de l'efficacité des produits de séparation détectés s'effectue par addition de cellules adhérentes.
